# EUROPÄISCHE PATENTSCHRIFT

(11) **EP 0 488 170 B1**
(45) Veröffentlichungstag und Bekanntmachung des Hinweises auf die Patenterteilung: **03.07.1996**
(21) Anmeldenummer: 91120187.9
(22) Anmeldetag: 26.11.1991
(51) Int. Cl.: G01N 33/68, G01N 33/566, G01N 33/543, C12N 15/62

(54) **Zellfreie Rezeptorbindungsteste, ihre Herstellung und Verwendung**
Cell-free receptor binding tests, their production and use
Nécessaire pour le dosage de liaisons à des récepteurs exempts de cellules, leurs production et utilisation

(30) Priorität: 28.11.1990 DE 4037837
(43) Veröffentlichungstag der Anmeldung: 03.06.1992
(73) Patentinhaber: BEHRINGWERKE Aktiengesellschaft, 35001 Marburg (DE)
(72) Erfinder: Lauffer, Leander, Dr., W-3550 Marburg (DE); Zettlmeissl, Gerd, Dr., W-3552 Wetter (DE); Oquendo, Patricia, Dr., W-3550 Marburg (DE)

(56) Entgegenhaltungen:
- EP-A- 0 244 221
- EP-A- 0 314 317
- EP-A- 0 325 262
- EP-A- 0 327 522
- WO-A-88/09344
- WO-A-91/17252

## Beschreibung

Die Erfindung betrifft zellfreie Rezeptorbindungsteste, die es erlauben, das Bindungsverhalten von zellmembranständigen Rezeptorproteinen gegenüber natürlichen oder künstlichen Liganden zu untersuchen. Dabei wird der jeweilige Rezeptor mit einem geeigneten Trägermolekül, vorzugsweise der schweren Kette eines Immunglobulins, verknüpft und über den Träger unter Erhalt seiner biologischen Eigenschaft an eine geeignete Festphase gebunden.

Für viele biologische und medizinische Fragestellungen ist die Bestimmung des Bindungsverhaltens von zellmembranständigen Rezeptorproteinen gegenüber natürlichen oder künstlichen Liganden von Bedeutung. Hierfür wird üblicherweise ein Ligand radioaktiv markiert und seine spezifische Bindung durch geeignete Methoden wie Gleichgewichtszentrifugation, Gleichgewichtsdialyse oder Filtration bestimmt. Die Rezeptormoleküle können hierbei zellgebunden bleiben, in welchem Fall der Test mit ganzen Zellen ausgeführt wird, können jedoch auch in subzellulären Fraktionen wie Membranvesikeln vorliegen oder auch mit geeigneten Detergentien aus der Zellmembran extrahiert und in Detergens-Mizellen stabilisiert werden. Die genannten Rezeptorbindungstests werden mit abnehmender Anzahl von Rezeptormolekülen in der Zellmembran zunehmend schwieriger durchzuführen. Viele Rezeptoren, insbesondere auch solche von großem medizinischen Interesse, werden normalerweise in nur geringem Ausmaß (in der Größenordnung von einigen Hundert bis einigen Tausend Molekülen pro Zelle) auf der Zelloberfläche exprimiert. Hierzu gehören z. B. die Rezeptoren für Granulocyten/Makrophagen-koloniestimulierender Faktor (GM-CSF), viele Interleukine, Erythropoietin, Tumornekrosefaktor (TNF) etc.

Bevorzugtes Ziel der vorliegenden Erfindung war es, geeingete Bindungsteste für vorzugsweise solche Rezeptoren zu entwickeln. Ein spezifischer, sensitiver und einfach durchzuführender Bindungstest für diese und andere Rezeptoren würde es z. B. ermöglichen, eine große Anzahl von Verbindungen auf ihre Bindungseigenschaften zu testen, um so Agonisten- oder Antagonistenkandidaten zu identifizieren ("Rezeptorscreening"). Darüber hinaus könnten mit solchen Testen auch Antikörper gerichtet gegen Rezeptoren oder Liganden daraufhin überprüft werden, ob sie gegen Epitope, die in der Bindung eine Rolle spielen, gerichtet sind. Erfindungsgemäß bestand die Aufgabe darin, ein solches Testsystem herzustellen. Aus der EP-A-0 327 522 ist ein diagnostischer Test bekannt, bei dem ein Antigen an ein serumalbumin-bindendes Polypeptidfragment fusioniert ist, welches an eine humanserumalbumin-bedeckte Oberfläche gebunden ist. Aus den EP-A 0325 262, EP-A 0314 317 und der deutschen Patentanmeldung (DE) P 4020 607.6 sind Fusionsproteine bestehend aus verschiedenen Anteilen der extrazellulären Domänen menschlicher Membranproteine oder löslicher Proteine (Fusionspartner) und dem konstanten Teil (Fc) der schweren Kette eines Ig bekannt bzw. vorgeschlagen. Hierbei wird auf DNA-Ebene die kodierende Sequenz des Fusionspartners mit einer für den Fc-Teil kodierenden DNA derart fusioniert, daß der Fusionspartner vorzugsweise den aminoterminalen Anteil des Fusionsproteins ausmacht. Die rekombinante DNA wird dann in geeigneten Zellsystemen exprimiert. Fusionspartner für den Fc-Teil sind einerseits zur Immunglobulinfamilie gehörige Proteine wie das T-Zellantigen CD4 (EP-A 0325 262 und 0314 317), andererseits auch strukturell nicht verwandte Proteine wie Thromboplastin oder der Rezeptor für Interleukin-4 (IL-4) (DE P 4020607.6). Die genannten Fusionsproteine werden vorzugsweise in Animalzellen exprimiert. Der aminoterminale Fusionspartner behält in der Regel seine biologische Aktivität; stammt er beispielsweise von einem normalerweise membranständigen Rezeptorprotein, bindet er Liganden mit einer Affinität, die der des membrangebundenen Rezeptors gleichkommt. Ist also ein Rezeptor-Protein auf cDNA-Ebene charakterisiert, besteht prinzipiell die Möglichkeit, in rekombinanten Expressionssystemen große Mengen biologisch aktiver Moleküle herzustellen. Diese können dann, wie unten beschrieben, in einem Bindungstest eingesetzt werden. In Animalzellen werden die beschriebenen Fusionsproteine sezerniert und können leicht affinitätschromatographisch aus dem Kulturüberstand gereinigt werden, da sie über ihren Fc-Teil an Protein A binden, das seinerseits an z. B. Sepharose® gekoppelt werden kann. Die Synthese der Fusionsproteine kann auch in bekannten prokaryontischen Expressionssystemen (E. coli, Pseudomonas, Bacillus etc.) bzw. Hefen (z. B. Saccharomyces cerevisiae) unter Verwendung bekannter geeigneter Expressionsvektoren erfolgen. Neben dem beschriebenen und für die Erfindung bevorzugten Fc-Teil (Träger) können Fusionspartner erfindungsgemäß auch an beliebige andere Trägerproteine (z. B. Albumin, Protein A, Protein G, Glutathion-S-Transferase, Staphylococcus aureus Nuklease) gekoppelt werden.

Zur Durchführung der erfindungsgemäßen Rezeptorbindungsteste wird zunächst ein gegen den Träger-Teil gerichtetes Antiserum (oder monoklonaler Antikörper) oder ein anderes geeignetes Agens zur Beschichtung einer Festphase, z. B. einer ELISA-Testplatte, verwendet. Da bevorzugt der Fc-Teil eines Antikörpers und besonders bevorzugt der Fc-Teil eines humanen IgG1 als Träger eingesetzt wird, wird vorzugsweise ein spezifisch gegen die CH2-Domäne eines menschlichen IgG1 gerichtetes Kaninchenserum verwendet. An die vorbeschichtete Festphase werden dann die Fusionsproteine über ihren Träger-Teil gebunden.

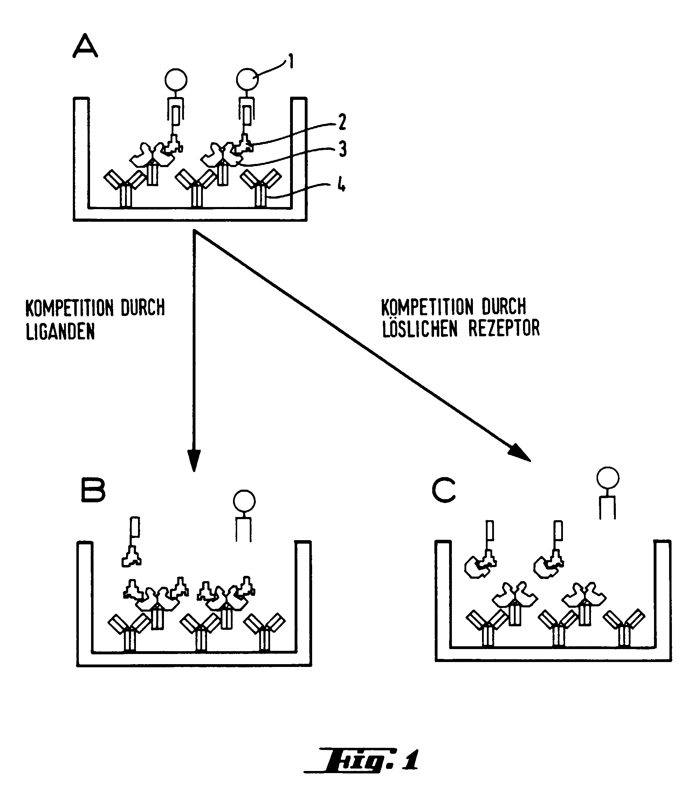

Somit bleiben die auf dem Rezeptoranteil lokalisierten Bindungsstellen für Liganden zugänglich. Um nun den gebundenen Liganden nachweisen zu können, muß dieser markiert sein. Dies kann z. B. durch Einführung eines radioaktiven Nuklids geschehen (z. B. Siekierka, J.J. und DeGudicibus, S., Anal. Biochem. Bd 172 (1988), 514-517), vorzugsweise jedoch durch Biotinylierung des Liganden (King und Catino, Anal.Biochem. Bd. 188 (1990), 97-100). Dieser wird dann seinerseits durch ein Streptavidin-Enzymkonjugat, vorzugsweise Streptavidin-Peroxidase, nachgewiesen. Der prinzipielle Aufbau der erfindungsgemäßen Teste ist in Fig. 1 beispielhaft dargestellt.

Die erfindungsgemäßen Teste haben breite Verwendungsmöglichkeiten, von denen die bevorzugten nachstehend aufgeführt sind und ebenfalls die Erfindung ausmachen:
- Verwendung im "Rezeptorscreening":
   a) Rezeptor in der Festphase: Screening einer großen Anzahl von Substanzen zur Identifizierung von Agonisten und Antagonisten des Liganden.
   b) Ligand in der Festphase: Screening einer großen Anzahl von Substanzen zur Identifizierung von Agonisten und Antagonisten des Rezeptors.
- Verwendung im Antikörperscreening:
   Identifizierung von Antikörpern, die die Wechselwirkung von Ligand und Rezeptor beeinflussen.
- Verwendung zum quantitativen Nachweis der Bindungsaktivität von löslichen Rezeptorformen.
- Verwendung zum quantitativen Nachweis der biologischen Aktivität spezificher Liganden.
- Funktionelle Analyse von veränderten Liganden ("Muteinen") oder Teilen (z. B. Oligopeptiden) davon.
- Identifizierung von Substanzen, die die Wechselwirkung pathogener Organismen (z. B. Viren oder Bakterien) mit ihren zellulären Rezeptoren beeinflussen.
- Identifizierung von Substanzen, die die Wechselwirkung zellulärer Adhäsionsmoleküle beeinflussen.

Die Erfindung ist ferner in den Beispielen näher erläutert und in den Patentansprüchen enthalten.

### Beispiel 1:

### Zellfreier IL-4 Bindungstest

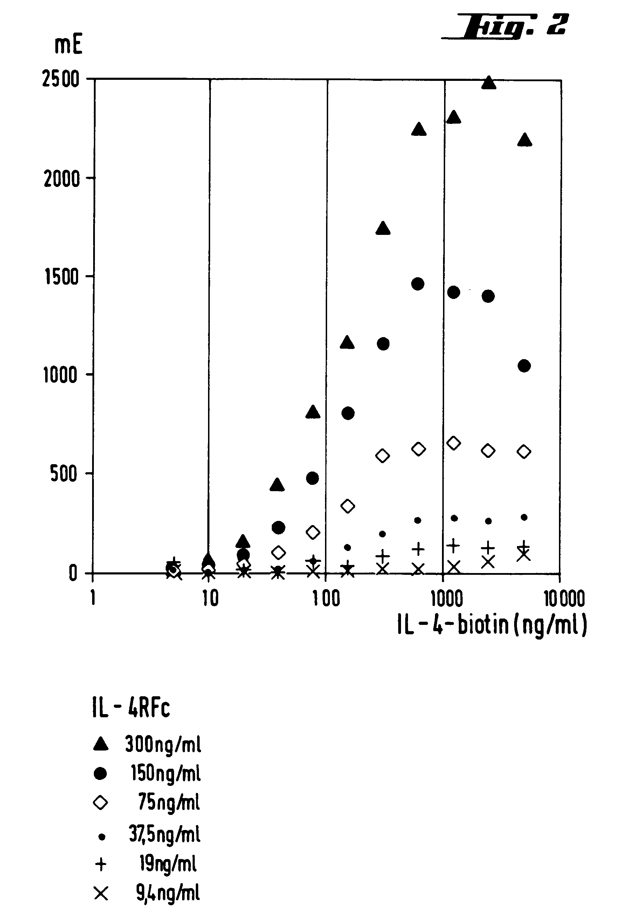

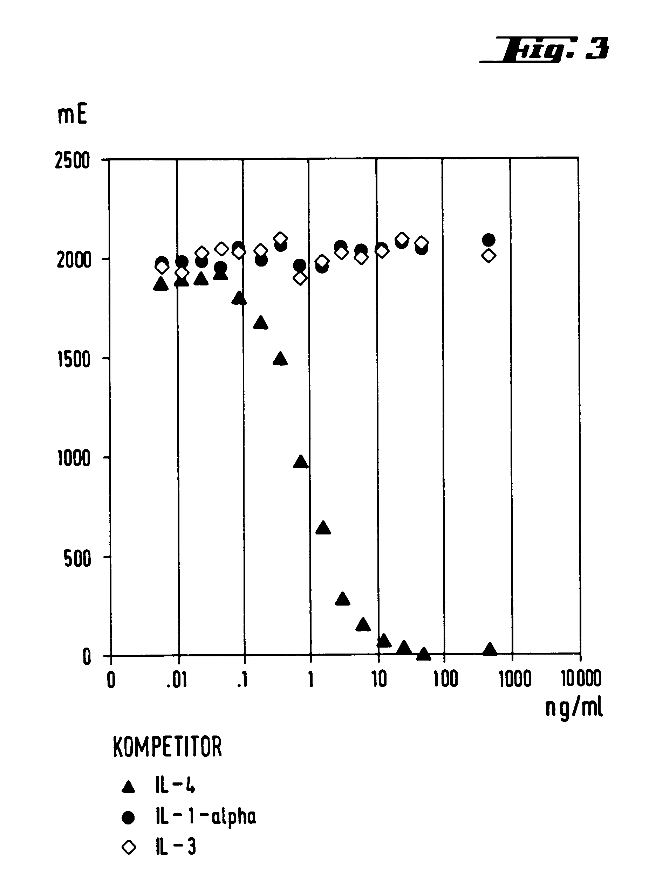

Aus der DE P 4020 607.6 ist das Protein IL-4RFc bekannt. Es besteht aus dem extrazellulären Anteil des menschlichen Rezeptors für IL-4, der an den Fc-Teil der schweren Kette eines humanen IgG1-Moleküls fusioniert ist. ELISA-Testplatten (Nunc, Typ B) wurden über Nacht bei 4°C mit 100 µl eines affinitätsgereinigten Kaninchenserums gegen die CH2-Domäne von menschlichem IgG (Dakopatts) beschichtet. Die Konzentration betrug 10 µg/ml in PBS (137 mM NaCl, 2.7 mM KCl, 6.1 mM Na2HPO4, 3.9 mM KH2P04, 0.5 mM MgC12, 0.1 mM CaC12, pH 7.0). Nach fünfmaligem Waschen mit PBS enthaltend 0.05 % Tween 20® wurde die Testplatte mit 275 µl PBS enthaltend 5 % Trockenmagermilch 60 min bei Raumtemperatur inkubiert und danach wie oben gewaschen. Es wurde dann zunächst eine "Schachbretttitration" vorgenommen, um eine geeignete Kombination von Rezeptor- und Ligandenkonzentrationen für den Aufbau des Tests zu bestimmen. Hierzu wurde die Testplatte zunächst mit verschiedenen Konzentrationen an gereinigtem IL-4RFc-Protein (100 µl) in Eagle's Medium (modifiziert nach Dulbecco) enthaltend 10 % fötales Kälberserum (DMEM/FKS) 60 min bei Raumtemperatur inkubiert und danach wie oben gewaschen. IL-4 wurde mittels N-Hydroxysuccinimid-Biotin (Sigma) biotinyliert (Niendorf, A. et al., J. Histochem. Cytochem. Bd. 34 (1986), 357--361) und in verschiedenen Konzentrationen in DMEM/FKS (100 µl) ebenfalls 60 min bei Raumtemperatur an die IL-4RFc-vorinkubierte Testplatte gebunden. Nach Waschen wie oben wurde 30 min bei Raumtemperatur mit 100 µl Streptavidin-Peroxidase (Amersham; 1:250 in DMEM/FKS) inkubiert und danach wie oben gewaschen. Der Nachweis der gebundenen Peroxidase geschah durch Farbentwicklung in 100 µl Tetramethylbenzidin-Substratlösung (Behringwerke). Nach 30 min Inkubation bei Raumtemperatur wurde die Extinktion bei 450 nm gemessen. Die Ergebnisse des Versuches sind in Fig. 2 dargestellt. Abhängig von den eingesetzten Mengen an Rezeptor und Ligand wird eine Anzahl typischer Bindungskurven erhalten. Das Signal, das den gebundenen Liganden reflektiert, erreicht einen Plateauwert, der umso höher liegt, je mehr Rezeptor zur Beschichtung der Testplatte eingesetzt wurde. Für weitergehende Versuche mit den in Fig. 1 schematisch dargestellten Kompetitionstests wurde eine Kombination von 300 ng/ml IL-4RFc zur Beschichtung und 300 ng/ml IL-4-Biotin für die Bindung verwendet. Zur Konzentrationsberechnung von IL-4-Biotin wie auch der in den Beispielen 3 und 5 aufgeführten Liganden wurde angenommen, daß aller zurBiotinvlierung eingesetzter Ligand auch tatsächlich zurückerhalten wurde. Die verwendete Kombination ergibt ein Signal (ca. 2000 mE), das (i) deutlich zu messen ist und (ii) noch vor Erreichen des Plateauwertes für die verwendete Rezeptorkonzentration liegt. Um Spezifität und Sensitivität des Tests zu bestimmen, wurde untersucht, inwieweit verschiedene Liganden mit IL-4-Biotin um die Bindung an IL-4RFc kompetieren können (Fig. 3). Hierzu wurde eine konstante Konzentration (300 ng/ml) IL-4-Biotin in Gegenwart verschiedener Konzentrationen an IL-1-alpha, IL-3 und IL-4 eingesetzt. Fig. 3 zeigt, daß nur IL-4 wirksam kompetieren kann, während IL-1-alpha und IL-3 in den eingesetzten Konzentrationen keine Bindungsaktivität zeigen. Die Konzentration an IL-4, bei der eine halbmaximale Inhibition der Bindung von IL-4-Biotin beobachtet wird (IC50), liegt bei 1 ng/ml.

### Beispiel 2:

### Nachweis löslichen IL-4 Rezeptors im zellfreien Bindungstest

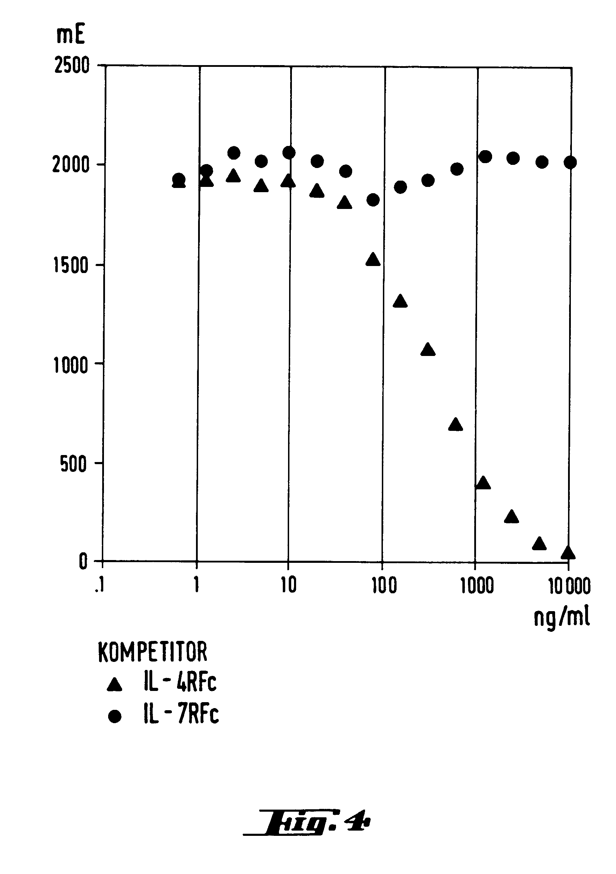

Es sollte gezeigt werden, ob mit dem Test nicht nur kompetierende Liganden nachgewiesen werden können, sondern auch rekombinante lösliche Formen von Rezeptoren. Hierzu wurde das IL-4RFc-Protein selbst gewählt. Der Testaufbau war wie in Beispiel 1 beschrieben, es wurden jedoch nach Inkubation der Testplatte mit der Standardkonzentration IL-4RFc (300 ng/ml) verbleibende freie Fc-Bindungsstellen durch Inkubation mit 10% Humanserum in DMEM/FKS (60 min bei Raumtemperatur) abgesättigt. Danach wurde der Bindungstest in Gegenwart verschiedener Konzentrationen von IL-4RFc durchgeführt. Fig. 4 zeigt, daß IL-4RFc in diesem Kompetitionstest tatsächlich nachgewiesen werden kann, während IL-7RFc, eine Fc-Fusion des humanen IL-7-Rezeptors, keine Bindungsaktivität aufweist. Die IC50 für IL-4RFc liegt bei 250 ng/ml.

### Beispiel 3:

### Zellfreier TNF Bindungstest

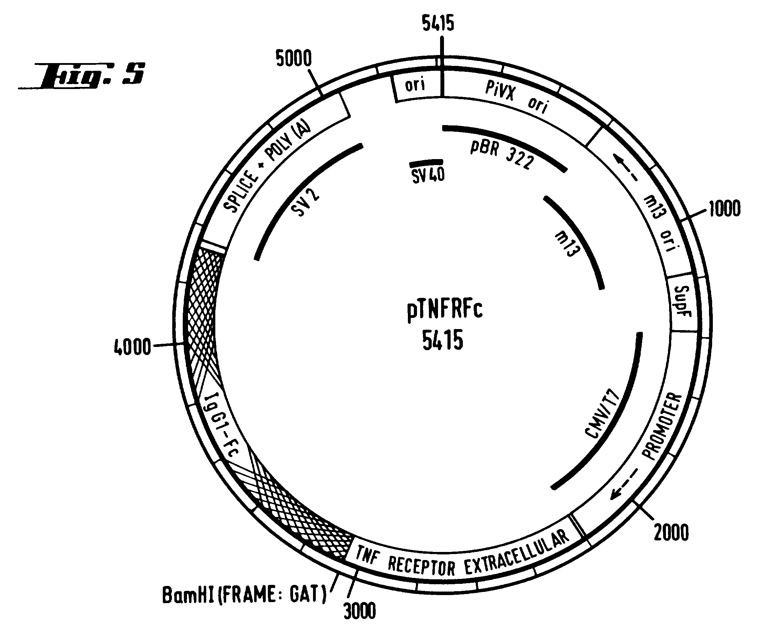

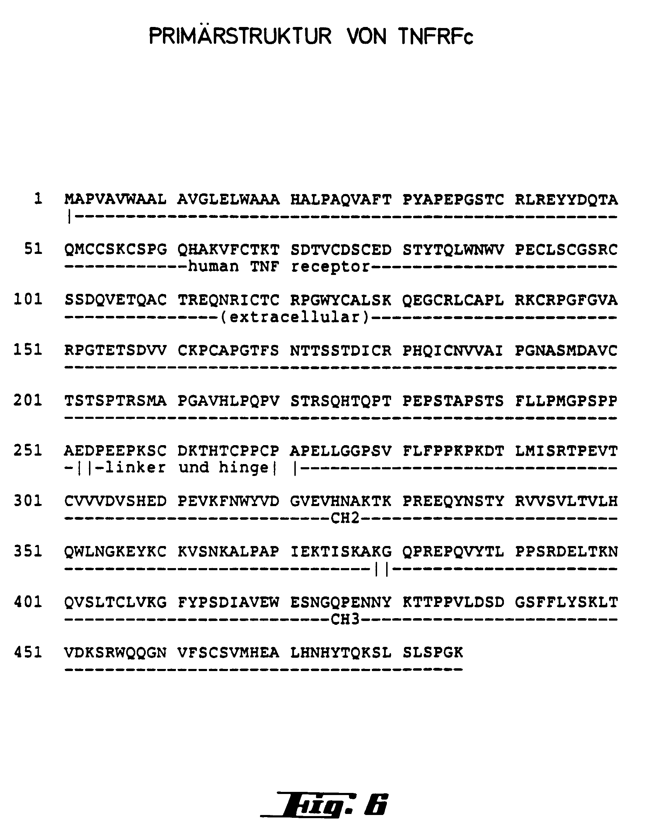

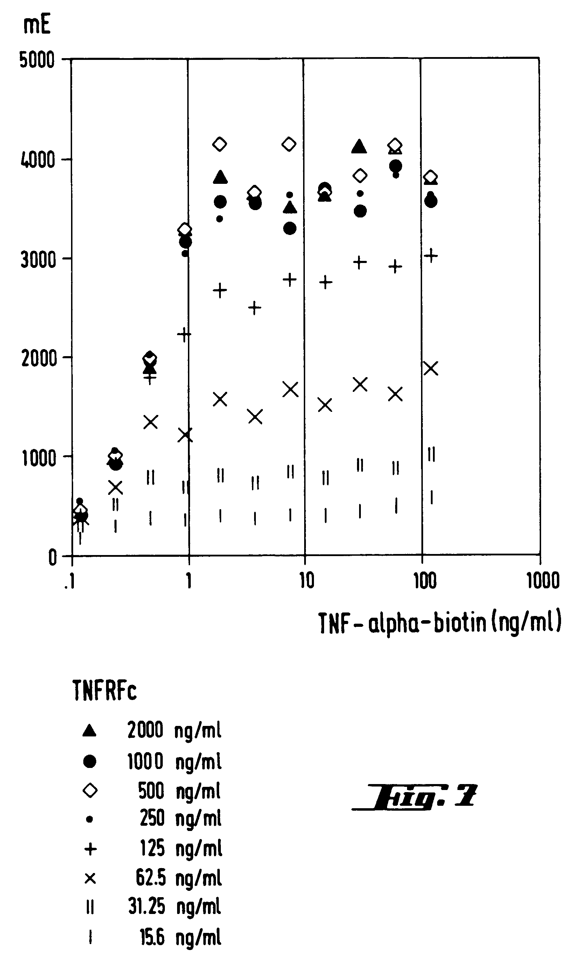

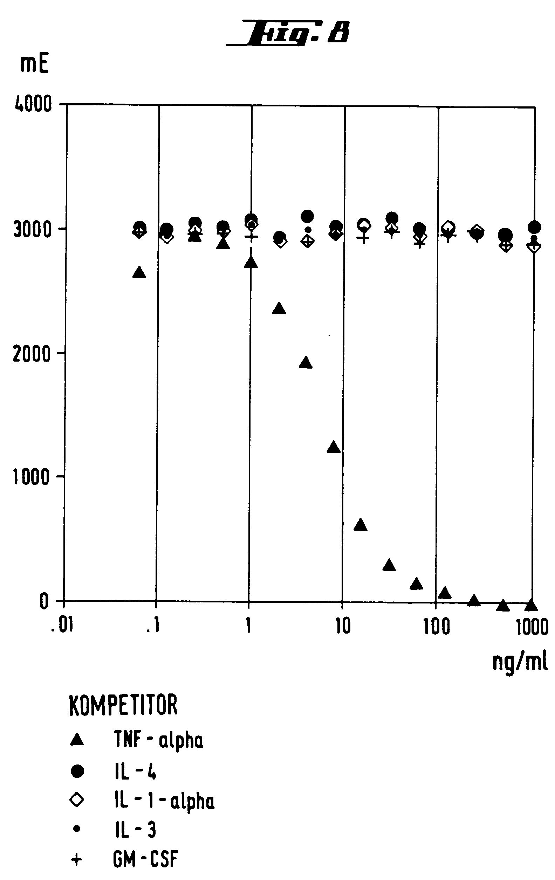

cDNA für die 80 kD-Form des menschlichen TNF-Rezeptors wurde kürzlich isoliert (Smith, C. A. et al., Science, Bd. 248 (1990), 1019-1023). Sie kodiert für ein typisches Membranprotein bestehend aus der aminoterminalen extrazellulären Domäne, einer Transmembranregion und einer carboxyterminalen cytoplasmatischen Domäne. In der kodierenden Region für den TNF-Rezeptor liegt direkt vor den Kodons für die letzten fünf Aminosäurereste der extrazellulären Region eine singuläre Schnittstelle für das Restriktionsenzym PvuII. Das die cDNA enthaltende Expressionsplasmid huTNFRcavnot wurde mit PvuII geschnitten und mit BamHI-Linkern (5' CGGATCCG 3') ligiert. Danach wurde mit NotI gespalten, das direkt vor der 5'-untranslatierten Region des TNF-Rezeptors schneidet. Der entstehende Überhang wurde mittels Klenow-Enzym aufgefüllt und danach eine Spaltung mit BamHI durchgeführt. Das erhaltene NotI (aufgefüllt)/BamHI-Fragment (∼800 bp) kodiert für die gesamte extrazelluläre Domäne des TNF-Rezeptors (mit Ausnahme der fünf Aminosäurereste direkt vor der Transmembranregion) mit einem durchgehenden Leserahmen vom Initiationskodon bis zu der in der BamHI-Erkennungssequenz enthaltenen Nukleotidfolge GAT, die in diesem Leserahmen für einen Asparaginsäurerest kodiert. Dieses Fragment wurde in den aus der DE P 4020607.6 bekannten Vektor p4EGammaH kloniert. Hierzu wurde p4EGammaH mit HindIII und nach Auffüllung des entstehenden Überhangs mittels Klenow-Enzym mit BamHI geschnitten. Das erhaltene Expressionsplasmid pTNFRFc kodiert für das Fusionsprotein TNFRFc aus dem extrazellulären Anteil des TNF-Rezeptors, der über die Hinge-Region an den Fc-Teil der schweren Kette eines menschlichen IgG1 gekoppelt ist. pTNFRFc ist schematisch in Fig. 5, die Aminosäuresequenz des darin kodierten TNFRFc in Fig. 6 dargestellt. pTNFRFc wurde in BHK-Zellen transfiziert und stabile Klone wurden nach Doppelselektion mit Methotrexat und G418 erhalten (EP-A 0330 977). Typische Expressionsraten lagen bei 20 µg/ml Überstand, aus dem TNFRFc mittels Chromatographie auf Protein A-Sepharose® isoliert wurde (DE P 4020607.6). Wie in Beispiel 1 beschrieben, wurde mit TNFRFc und biotinyliertem TNF-alpha zunächst eine Schachbrettitration vorgenommen, um geeignete Bedingungen für einen Bindungstest zu definieren. Fig.7 zeigt eine Serie von Bindungskurven, die in diesem Versuch erhalten wurden. Für weitere Versuche wurde die Kombination von 200 ng/ml TNFRFc zur Beschichtung und 20 ng/ml TNF-alpha-Biotin für die Bindunq gewählt. Auch der TNF-Bindungstest ist sensitiv und spezifisch für TNF-alpha. In Fig. 8 ist das Ergebnis eines Kompetitionsversuch dargestellt. Nur TNF-alpha hemmt Bindung von TNF-alpha-Biotin mit einer IC50 von 5 ng/ml, während IL-1-alpha, IL-3, IL-4 und GM-CSF in den verwendeten Konzentrationen keinen Effekt zeigen.

### Beispiel 4:

### Nachweis löslichen TNF-Rezeptors im zellfreien Bindungstest

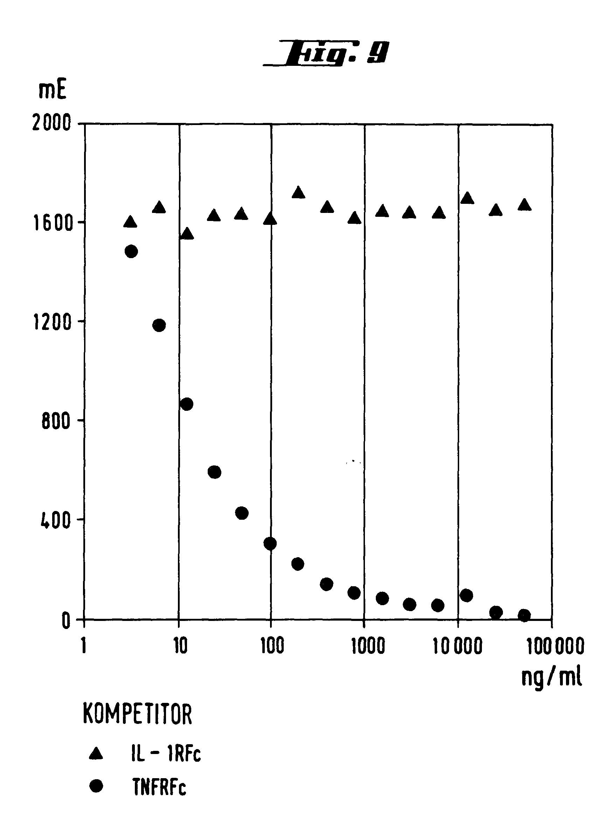

Dieser Versuch wurde analog zu dem im Beispiel 2 beschriebenen durchgeführt (Fig. 9). TNFRFc hemmt mit einer IC50 von 35 ng/ml, während IL-1RFc, ein Fusionsprotein aus dem humanen IL-1-Rezeptor und dem Fc-Teil eines menschlichen IgG1, nicht inhibiert.

### Beispiel 5:

### Zellfreier GM-CSF Bindungstest

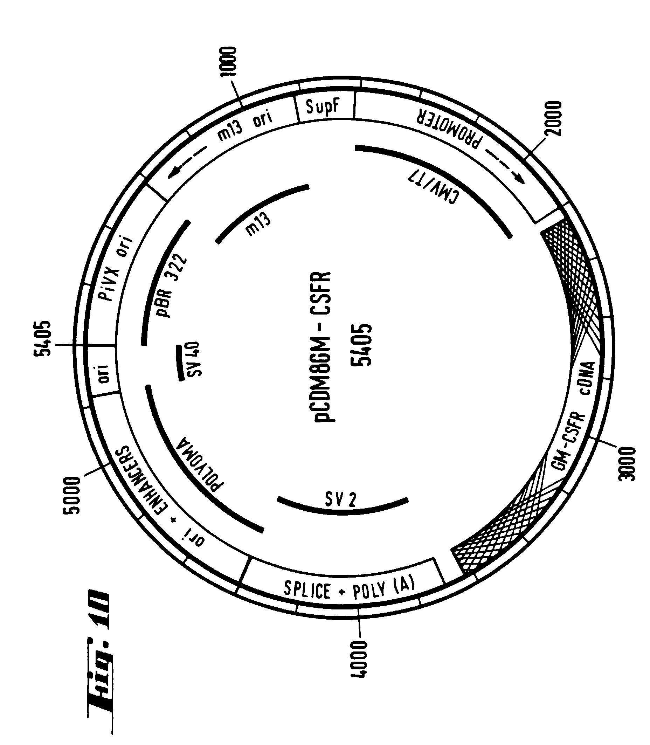

cDNA für einen humanen Rezeptor für GM-CSF wurde isoliert (Gearing, D.P. et al., EMBO J. Bd. 8 (1989), 3667-3676). Auch der GM-CSF-Rezeptor ist ein typisches Membranprotein mit aminoterminaler extrazellulärer Domäne, Transmembranregion und carboxyterminaler intrazellulärer Domäne. Zwei Oligonukleotide wurden synthetisiert, die mit Regionen in der 5'-untranslatierten Region (Oligonukleotid A: 5'AGCAGGTGGAAGGAGAGGAAGCGG 3') bzw. 3'-untranslatierten Region (Oligonukleotid B:
5'AAGAATGGGAACAGGCAGGCCTGGGC 3') hybridisieren können. Amplifizierung der Plasmid-DNA einer cDNA-Genbank von humaner Plazenta (Simmons, D. und Seed, B., Nature Bd. 333 (1988), 568-570) mit thermostabiler Taq-DNA-Polymerase ergab ein DNA-Fragment (ca. 1400 bp), das in seiner Größe der GM-CSF-Rezeptor-cDNA entspricht. Restriktionsanalysen bestätigten die Identität des amplifizierten DNA-Fragmentes. Nach Ligierung von BstXI-Adaptern (Aruffo, A. und Seed, B., Proc. Natl. Acad. Sci. USA Bd. 84, 8573-8577) wurde das Fragment in den BstXI-behandelten eukaryontischen Expressionsvektor CDM8 (Seed, B., Nature Bd. 329 (1987), 840-842) eingesetzt. Das resultierende Plasmid pCDM8GM-CSFR ist schematisch in Fig. 10 dargestellt.

Um eine Fc-Fusion mit GM-CSF-Rezeptor herstellen zu können, wurde eine erneute DNA-Amplifizierung mit thermostabiler Taq-DNA-Polymerase an pCDM8 durchgeführt.

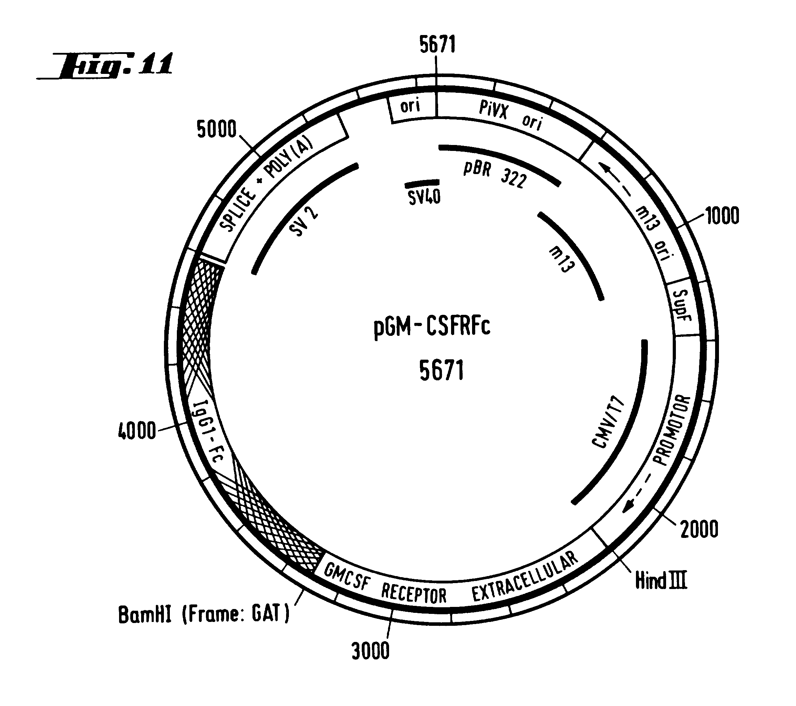

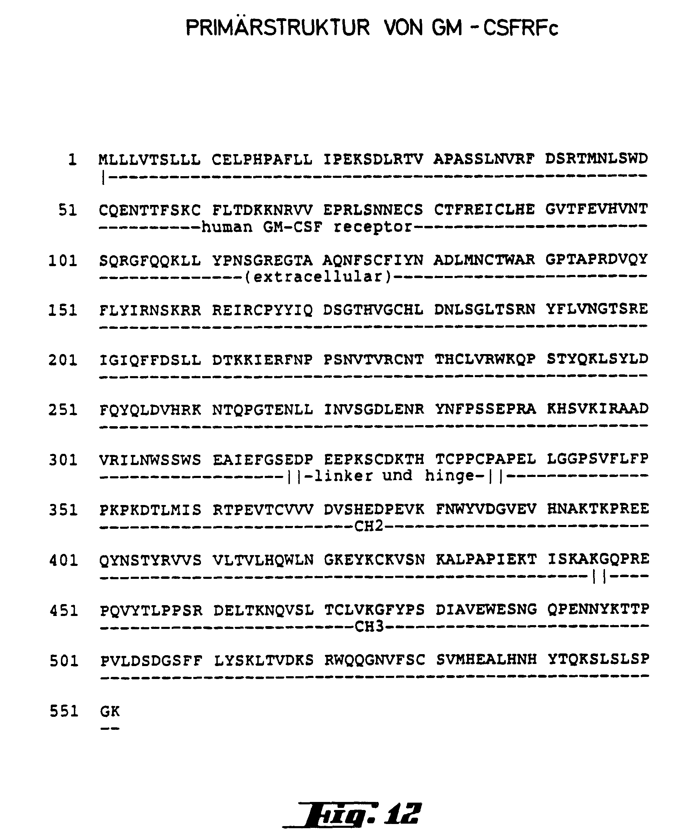

Hierzu wurden zwei weitere Oligonukleotide synthetisiert. Oligonukleotid C (5'GATCGATTAAGCTTAGCAGGTGGAAGGAGAGGAAGCGGATGCCG 3') hybridisiert mit der 5'-untranslatierten Region und führt vor dieser eine HindIII-Schnittstelle ein, Oligonukleotid D (5'GCCATTGAATTTGGTTCTGAGGATCCAGATATGC 3') hybridisiert mit der cDNA vor der kodierenden Region für die Transmembrandomäne und führt direkt vor dieser eine BamHI-Schnittstelle ein. Das erwartete 1131 bp-Fragment wurde erhalten und nach Behandlung mit BamHI und HindIII in den BamHI/HindIII-geschnittenen Vektor p4EGammaH eingeführt. Das so erzeugte Expressionsplasmid pGM-CSFRFc kodiert für das Fusionsprotein GM-CSFRFc bestehend aus der extrazellulären Domäne des GM-CSF-Rezeptors, die über die Hinge-Region an den Fc-Teil eines menschlichen IgG1 gekoppelt ist. pGM-CSFRFc und die Peptidsequenz des Fusionsproteins GM-CSFRFc sind in Fig. 11 bzw. 12 dargestellt.

pGM -CSFRFc wurde transient in COS-Zellen exprimiert. Hierzu wurden COS-Zellen mit Hilfe von DEAE-Dextran mit pGM-CSFRFc transfiziert (EP-A 0325 262). Die Überstände wurden für den zellfreien GM-CSF-Bindungstest verwendet.

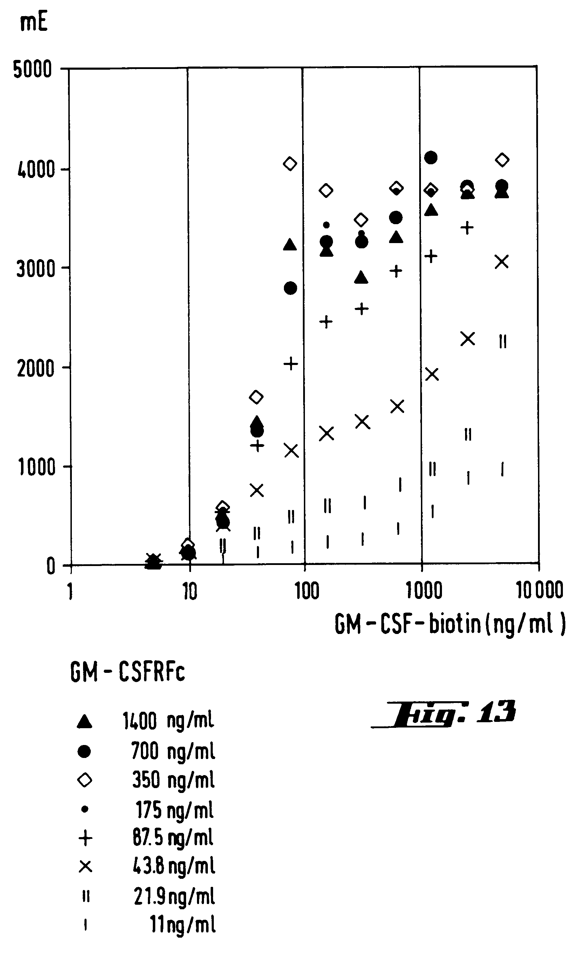

Fig. 13 zeigt das Ergebnis der Schachbrettitration mit GM-CSFRFc und biotinyliertem GM-CSF. Für weitere Versuche wurde die Kombination 1000 ng/ml GM-CSFRFc zur Beschichtung und 150 ng/ml GM-CSF-Biotin für die Bindung gewählt.

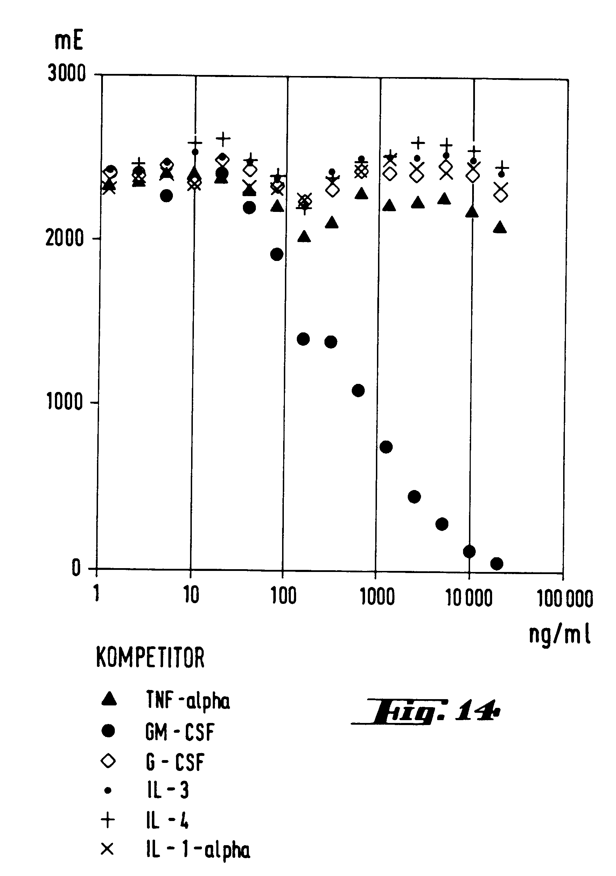

Der Bindungstest ist spezifisch für GM-CSF. Unter den gewählten Bedingungen hemmt unmarkiertes GM-CSF die Bindung von GM-CSF-Biotin mit einer IC50 von 200 ng/ml, während TNF-alpha, G-CSF, IL-1-alpha, IL-3 und IL-4 in den verwendeten Konzentrationen nicht inhibieren (Fig.14).

### Beispiel 6:

### Charakterisierung von monoklonalen Antikörpern gegen GM-CSF im zellfreien Rezeptorbindungstest

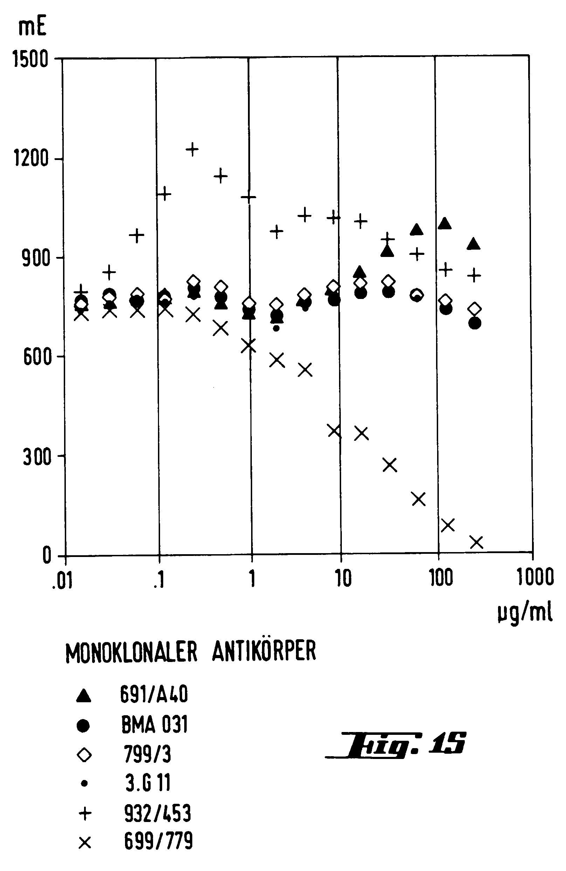

Es wurde eine Reihe verschiedener monoklonaler Antikörper gegen GM-CSF hergestellt (Behringwerke). Sie wurden gleichzeitig mit GM-CSF-Biotin im Bindungstest eingesetzt unter den in Beispiel 5 beschriebenen Bedingungen (Fig. 15).

Der monoklonale Antikörper 699/779 hemmt die Bindung, ist also mit hoher Wahrscheinlichkeit gegen ein Rezeptor-Bindungsepitop auf GM-CSF gerichtet. Die monoklonalen Antikörper 691/A40, 799/3, 3.G11, 932/453 sowie der Kontrollantikörper BMA031 (gerichtet gegen einen humanen T-Zellrezeptor) hingegen inhibieren nicht. 932/453 und in geringerem Maße auch 691/A40 bewirken sogar eine Erhöhung des gemessenen Signals.

### Legenden zu den Abbildungen

Legende zu Fig. 1:
Prinzipieller Aufbau des zellfreien Rezeptorbindungstests:
A) Ein Rezeptor/Träger-Fusionsprotein (3) wird über einen Anti-Träger-Antikörper (4) oder ein anderes geeignetes Agens an eine Festphase gebunden. An die RezeptorBindungsstelle kann ein markierter Ligand (2) binden, der seinerseits über ein Nachweis/Amplifikationssystem (1) gemessen werden kann.
B) Ein unmarkierter Ligand des Rezeptors kann über die Kompetition für die Rezeptorbindungsstelle nachgewiesen werden.
C) Löslicher Rezeptor kann über die Kompetition für den markierten Liganden nachgewiesen werden.

Legende zu Fig. 2:
Zellfreier IL-4-Bindungstest: Abhängigkeit der IL-4-Biotin-Bindung von der zur Beschichtung der ELISA-Testplatte verwendeten IL-4RFc- Konzentration.

Legende zu Fig. 3:
Zellfreier IL-4-Bindungstest:
Abhängigkeit der IL-4-Biotin-Bindung von der Konzentration der zur Kompetition eingesetzten Liganden.

Legende zu Fig. 4:
Zellfreier IL-4-Bindungstest:
Abhängigkeit der IL-4-Biotin-Bindung von der Konzentration der zur Kompetition eingesetzten Rezeptor/Fc-Fusionsproteine.

Legende zu Fig. 5:
Schematischer Aufbau des Expressionsplasmids pTNFRFc.

Legende zu Fig. 6:
Aminosäuresequenz des Fusionsproteins TNFRFc.

Legende zu Fig. 7:
Zellfreier TNF-alpha-Bindungstest:
Abhängigkeit der TNF-alpha-Biotin-Bindung von der zur Beschichtung der ELISA-Testplatte verwendeten TNFRFc-Konzentration.

Legende zu Fig. 8:
Zellfreier TNF-alpha-Bindungstest:
Abhängigkeit der TNF-alpha-Biotin-Bindung von der Konzentration der zur Kompetition eingesetzten Liganden.

Legende zu Fig. 9:
Zellfreier TNF-alpha-Bindungstest:
Abhängigkeit der TNF-alpha-Biotin-Bindung von der Konzentration der zur Kompetition eingesetzten Rezeptor/Fc-Fusionsproteine.

Legende zu Fig. 10:
Schematischer Aufbau des Expressionsplasmids pCDM8GM-CSFR.

Legende zu Fig. 11:
Schematischer Aufbau des Expressionsplasmids pGM-CSFRFc.

Legende zu Fig. 12:
Aminosäuresequenz des Fusionsproteins GM-CSFRFc.

Legende zu Fig. 13:
Zellfreier GM-CSF-Bindungstest:
Abhängigkeit der GM-CSF-Biotin-Bindung von der zur Beschichtung der ELISA-Testplatte verwendeten GM-CSFRFc-Konzentration.

Legende zu Fig. 14:
Zellfreier GM-CSF-Bindungstest:
Abhängigkeit der GM-CSF-Biotin-Bindung von der Konzentration der zur Kompetition eingesetzten Liganden.

Legende zu Fig. 15:
Zellfreier GM-CSF-Bindungstest:
Abhängigkeit der GM-CSF-Biotin-Bindung von der Konzentration der im Bindungstest eingesetzten anti-GM-CSF-Antikörper.

## Patentansprüche (Patentansprüche für folgende(n) Vertragsstaat(en): AT, BE, CH, DE, DK, FR, GB, IT, LI, LU, NL, SE)

1. Zellfreier Bindungstest zur Untersuchung des Bindungsverhaltens von normalerweise zellmembranständigen Rezeptorproteinen gegenüber natürlichen oder künstlichen Liganden, enthaltend
(1) einen Bindungspartner I, der ein rekombinantes Fusionsprotein aus einem für die zu untersuchende Bindung relevanten Rezeptorprotein und einem Trägerprotein ist, wobei besagtes Fusionsprotein mittels eines Antiserums oder monoklonalen Antikörpers unter Beibehaltung der Bindungsaktivität besagten Rezeptorproteins an eine geeignete Festphase gekoppelt ist, und
(2) einen Bindungspartner II, der ein in einer für die Messung der Bindung an besagtes Rezeptorprotein geeigneten Weise markierter Ligand für besagtes Rezeptorprotein, jedoch kein Antikörper ist.

2. Bindungstest nach Anspruch 1, in dem das Rezeptorprotein im rekombinanten Fusionsprotein die extrazelluläre Domäne eines zellulären Rezeptors oder ein Teil davon ist.

3. Bindungstest nach Anspruch 2, wobei der zelluläre Rezeptor ein Cytokinrezeptor ist.

4. Bindungstest nach Anspruch 2, wobei der zelluläre Rezeptor ein Wachstumsfaktorrezeptor ist.

5. Bindungstest nach Anspruch 2, wobei der zelluläre Rezeptor ein Hormonrezeptor ist.

6. Bindungstest nach Anspruch 2, wobei der zelluläre Rezeptor ein Neurotransmitterrezeptor ist.

7. Bindungstest nach Anspruch 2, wobei der zelluläre Rezeptor der Rezeptor für einen pathogenen Organismus oder ein Teil davon ist.

8. Bindungstest nach Anspruch 1, in dem das Rezeptorprotein im rekombinanten Fusionsprotein die extrazelluläre Domäne eines Zelladhäsionsmoleküls oder ein Teil davon ist.

9. Bindungstest nach Anspruch 1, in dem das Rezeptorprotein im rekombinanten Fusionsprotein ein lösliches Protein oder ein Teil davon ist.

10. Bindungstest nach Anspruch 1, in dem das Trägerprotein im rekombinanten Fusionsprotein ein Immunglobulinmolekül oder ein Teil davon ist.

11. Bindungstest nach Anspruch 10, in dem das Trägerprotein im rekombinanten Fusionsprotein der konstante Teil der schweren Kette eines Immunglobulins oder ein Teil davon ist.

12. Bindungstest nach Anspruch 10 oder 11, in dem das Trägerprotein im rekombinanten Fusionsprotein der konstante Teil der schweren Kette von humanem IgG 1 oder ein Teil davon ist.

13. Bindungstest nach Anspruch 1 unter Verwendung eines Trägerproteins im rekombinanten Fusionsprotein, worin das Trägerprotein die biologische Aktivität des Rezeptorproteins nicht beeinflußt und das Fusionsprotein einer Reinigung über Affinitätschromatographie zugänglich macht.

14. Bindungstest nach Ansprüchen 1 bis 13, in dem der Bindungspartner II radioaktiv markiert ist.

15. Bindungstest nach einem der Ansprüche 1 bis 13, in dem der Bindungspartner II mit einem niedermolekularen Liganden (wie z.B. Biotin) verknüpft wird und so in einem weiteren Reaktionsschritt nachgewiesen werden kann.

16. Bindungstest nach einem der Ansprüche 1 bis 13, in dem der Bindungspartner II mit einem enzymatisch aktiven Protein (z.B. Peroxidase, Alkalische Phosphatase, Luciferase) verknüpft ist.

17. Bindungstest nach Anspruch 15, in dem für den weiteren Reaktionsschritt an Peroxidase gekoppeltes Streptavidin verwendet wird.

18. Verwendung des Bindungstests nach einem der Ansprüche 1 bis 17 für Rezeptorscreening zur Identifizierung natürlicher oder synthetischer Agonisten oder Antagonisten der jeweiligen Wechselwirkung.

19. Verwendung des Bindungstests nach einem der Ansprüche 1 bis 17 zur Charakterisierung von Antikörpern gerichtet gegen einen der beiden Bindungspartner.

20. Verwendung des Bindungstests nach einem der Ansprüche 1 bis 17 zum Nachweis der biologischen Aktivität löslicher zellulärer Rezeptoren.

21. Verwendung des Bindungstests nach einem der Ansprüche 1 bis 17 zum Nachweis der biologischen Aktivität von Liganden.

22. Verwendung des Bindungstests nach einem der Ansprüche 1 bis 17 zur funktionellen Analyse gezielt veränderter Liganden oder Teilen davon.

23. Verwendung des Bindungstests nach einem der Ansprüche 1 bis 17 zur Identifizierung von diagnostisch oder therapeutisch relevanten Substanzen.

24. Verfahren zum Nachweis des Bindungsverhaltens normalerweise zellmembranständiger Rezeptorproteine gegenüber natürlichen oder künstlichen Liganden in einem zellfreien Bindungstest, dadurch gekennzeichnet, daß
(1) ein Bindungspartner I, der ein rekombinantes Fusionsprotein aus einem für die zu untersuchende Bindung relevanten Rezeptorprotein und einem Trägerprotein ist, wobei besagtes Fusionsprotein mittels eines Antiserums oder monoklonalen Antikörpers unter Beibehaltung der Bindungsaktivität besagten Rezeptorproteins an eine geeignete Festphase gekoppelt ist, an
(a) einen Bindungspartner II, der ein in einer für die Messung der Bindung an besagtes Rezeptorprotein geeigneten Weise markierter Ligand für besagtes Rezeptorprotein, jedoch kein Antikörper ist und
(b) einen zu bestimmenden nichtmarkierten Liganden für besagtes Rezeptorprotein oder ein zu bestimmendes Rezeptorprotein in löslicher Form
gebunden wird und
(2) die Menge an festphasengebundenem markierten natürlichen oder künstlichen Liganden nachgewiesen wird.

25. Verfahren nach Anspruch 24, dadurch gekennzeichnet, daß der zu bestimmende nichtmarkierte Ligand oder das zu bestimmende lösliche Rezeptorprotein ein Antikörper ist.

26. Verfahren zur Herstellung eines Bindungstestes nach einem der Ansprüche 1 bis 17, dadurch gekennzeichnet, daß ein rekombinantes Fusionsprotein (Bindungspartner I) aus einem für die zu untersuchende Bindung relevanten Rezeptorprotein und einem Trägerprotein mittels eines Antiserums oder monoklonalen Antikörpers, unter Beibehaltung der Bindungsaktivität des Rezeptorproteins an eine geeignete Festphase gekoppelt wird, wobei der andere Bindungspartner II, der kein Antikörper ist, in einer für die Messung der Bindung geeigneten Weise markiert wird.

## Patentansprüche (Patentansprüche für folgende(n) Vertragsstaat(en): ES, GR)

1. Verfahren zur Herstellung eines zellfreien Bindungstestes, dadurch gekennzeichnet, daß ein rekombinantes Fusionsprotein (Bindungspartner I) aus einem für die zu untersuchende Bindung relevanten Rezeptorprotein und einem Trägerprotein mittels eines Antiserums oder monoklonalen Antikörpers, unter Beibehaltung der Bindungsaktivität des Rezeptorproteins an eine geeignete Festphase gekoppelt wird, wobei ein anderer Bindungspartner II, der ein Ligand für besagtes Rezeptorprotein, jedoch kein Antikörper ist, in einer für die Messung der Bindung an besagtes Rezeptorprotein geeigneten Weise markiert wird.

2. Verfahren nach Anspruch 1, in dem das Rezeptorprotein im rekombinanten Fusionsprotein die extrazelluläre Domäne eines zellulären Rezeptors oder ein Teil davon ist.

3. Verfahren nach Anspruch 2, wobei der zelluläre Rezeptor ein Cytokinrezeptor ist.

4. Verfahren nach Anspruch 2, wobei der zelluläre Rezeptor ein Wachstumsfaktorrezeptor ist.

5. Verfahren nach Anspruch 2, wobei der zelluläre Rezeptor ein Hormonrezeptor ist.

6. Verfahren nach Anspruch 2, wobei der zelluläre Rezeptor ein Neurotransmitterrezeptor ist.

7. Verfahren nach Anspruch 2, wobei der zelluläre Rezeptor der Rezeptor für einen pathogenen Organismus oder ein Teil davon ist.

8. Verfahren nach Anspruch 1, in dem das Rezeptorprotein im rekombinanten Fusionsprotein die extrazelluläre Domäne eines Zelladhäsionsmoleküls oder ein Teil davon ist.

9. Verfahren nach Anspruch 1, in dem das Rezeptorprotein im rekombinanten Fusionsprotein ein lösliches Protein oder ein Teil davon ist.

10. Verfahren nach Anspruch 1, in dem das Trägerprotein im rekombinanten Fusionsprotein ein Immunglobulinmolekül oder ein Teil davon ist.

11. Verfahren nach Anspruch 10, in dem das Trägerprotein im rekombinanten Fusionsprotein der konstante Teil der schweren Kette eines Immunglobulins oder ein Teil davon ist.

12. Verfahren nach Anspruch 10 oder 11, in dem das Trägerprotein im rekombinanten Fusionsprotein der konstante Teil der schweren Kette von humanem IgG 1 oder ein Teil davon ist.

13. Verfahren nach Anspruch 1 unter Verwendung eines Trägerproteins im rekombinanten Fusionsprotein, worin das Trägerprotein die biologische Aktivität des Rezeptorproteins nicht beeinflußt und das Fusionsprotein einer Reinigung über Affinitätschromatographie zugänglich macht.

14. Verfahren nach Ansprüchen 1 bis 13, in dem der Bindungspartner II radioaktiv markiert ist.

15. Verfahren nach einem der Ansprüche 1 bis 13, in dem der Bindungspartner II mit einem niedermolekularen Liganden (wie z.B. Biotin) verknüpft wird und so in einem weiteren Reaktionsschritt nachgewiesen werden kann.

16. Verfahren nach einem der Ansprüche 1 bis 13, in dem der Bindungspartner II mit einem enzymatisch aktiven Protein (z.B. Peroxidase, Alkalische Phosphatase, Luciferase) verknüpft ist.

17. Verfahren nach Anspruch 15, in dem für den weiteren Reaktionsschritt an Peroxidase gekoppeltes Streptavidin verwendet wird.

18. Verwendung des Bindungstests nach einem der Ansprüche 1 bis 17 für Rezeptorscreening zur Identifizierung natürlicher oder synthetischer Agonisten oder Antagonisten der jeweiligen Wechselwirkung.

19. Verwendung des Bindungstests nach einem der Ansprüche 1 bis 17 zur Charakterisierung von Antikörpern gerichtet gegen einen der beiden Bindungspartner.

20. Verwendung des Bindungstests nach einem der Ansprüche 1 bis 17 zum Nachweis der biologischen Aktivität löslicher zellulärer Rezeptoren.

21. Verwendung des Bindungstests nach einem der Ansprüche 1 bis 17 zum Nachweis der biologischen Aktivität von Liganden.

22. Verwendung des Bindungstests nach einem der Ansprüche 1 bis 17 zur funktionellen Analyse gezielt veränderter Liganden oder Teilen davon.

23. Verwendung des Bindungstests nach einem der Ansprüche 1 bis 17 zur Identifizierung von diagnostisch oder therapeutisch relevanten Substanzen.

24. Verfahren zum Nachweis des Bindungsverhaltens normalerweise zellmembranständiger Rezeptorproteine gegenüber natürlichen oder künstlichen Liganden in einem zellfreien Bindungstest, dadurch gekennzeichnet, daß
(1) ein Bindungspartner I, der ein rekombinantes Fusionsprotein aus einem für die zu untersuchende Bindung relevanten Rezeptorprotein und einem Trägerprotein ist, wobei besagtes Fusionsprotein mittels eines Antiserums oder monoklonalen Antikörpers unter Beibehaltung der Bindungsaktivität besagten Rezeptorproteins an eine geeignete Festphase gekoppelt ist, an
(a) einen Bindungspartner II, der ein in einer für die Messung der Bindung an besagtes Rezeptorprotein geeigneten Weise markierter Ligand für besagtes Rezeptorprotein, jedoch kein Antikörper ist und
(b) einen zu bestimmenden nichtmarkierten Liganden für besagtes Rezeptorprotein oder ein zu bestimmendes Rezeptorprotein in löslicher Form
gebunden wird und
(2) die Menge an festphasengebundenem markierten natürlichen oder künstlichen Liganden nachgewiesen wird.

25. Verfahren nach Anspruch 24, dadurch gekennzeichnet, daß der zu bestimmende nichtmarkierte Ligand oder das zu bestimmende lösliche Rezeptorprotein ein Antikörper ist.

## Claims (Claims for the following Contracting State(s): AT, BE, CH, DE, DK, FR, GB, IT, LI, LU, NL, SE)

1. A cell-free binding assay for investigating the binding behavior of receptor proteins, which are normally in the cell membrane, toward natural or artificial ligands, comprising
(1) a binding partner I which is a recombinant fusion protein composed of a receptor protein relevant for the binding to be investigated and of a carrier protein, where said fusion protein is coupled by means of an antiserum or monoclonal antibody, with retention of the binding activity of said receptor protein, to a suitable solid phase, and
(2) a binding partner II which is a ligand, which is labeled in a manner suitable for measuring the binding to said receptor protein, for said receptor protein but is not an antibody.

2. The binding assay as claimed in claim 1, in which the receptor protein in the recombinant fusion protein is the extracellular domain of a cellular receptor or a part thereof.

3. The binding assay as claimed in claim 2, wherein the cellular receptor is a cytokine receptor.

4. The binding assay as claimed in claim 2, where the cellular receptor is a growth factor receptor.

5. The binding assay as claimed in claim 2, where the cellular receptor is a hormone receptor.

6. The binding assay as claimed in claim 2, where the cellular receptor is a neurotransmitter receptor.

7. The binding assay as claimed in claim 2, where the cellular receptor is the receptor for a pathogenic organism or a part thereof.

8. The binding assay as claimed in claim 1, in which the receptor protein in the recombinant fusion protein is the extracellular domain of a cell adhesion molecule or part thereof.

9. The binding assay as claimed in claim 1, in which the receptor protein in the recombinant fusion protein is a soluble protein or part thereof.

10. The binding assay as claimed in claim 1, in which the carrier protein in the recombinant fusion protein is an immunoglobulin molecule or part thereof.

11. The binding assay as claimed in claim 10, in which the carrier protein in the recombinant fusion protein is the constant part of the heavy chain of an immunoglobulin or part thereof.

12. The binding assay as claimed in claim 10 or 11, in which the carrier protein in the recombinant fusion protein is the constant part of the heavy chain of human IgGl or part thereof.

13. The binding assay as claimed in claim 1 using a carrier protein in the recombinant fusion protein, in which the carrier protein does not influence the biological activity of the receptor protein and makes the fusion protein amenable to purification by affinity chromatography.

14. The binding assay as claimed in any of claims 1 to 13, in which the binding partner II is radiolabeled.

15. The binding assay as claimed in any of claims 1 to 13, in which the binding partner II is linked to a low molecular weight ligand (such as, for example, biotin) and can thus be detected in a further reaction step.

16. The binding assay as claimed in any of claims 1 to 13, in which the binding partner II is linked to an enzymatically active protein (for example peroxidase, alkaline phosphatase, luciferase).

17. The binding assay as claimed in claim 15, in which streptavidin coupled to peroxidase is used for the further reaction step.

18. The use of the binding assay as claimed in any of claims 1 to 17 for receptor screening for the identification of natural or synthetic agonists or antagonists of the particular interaction.

19. The use of the binding assay as claimed in any of claims 1 to 17 for the characterization of antibodies directed against one of the two binding partners.

20. The use of the binding assay as claimed in any of claims 1 to 17 for detecting the biological activity of soluble cellular receptors.

21. The use of the binding assay as claimed in any of claims 1 to 17 for detecting the biological activity of ligands.

22. The use of the binding assay as claimed in any of claims 1 to 17 for the functional analysis of ligands with specific modifications, or parts thereof.

23. The use of the binding assay as claimed in any of claims 1 to 17 for the identification of substances relevant to diagnosis or therapy.

24. A method for detecting the binding behavior of receptor proteins, which are normally in the cell membrane, toward natural or synthetic ligands in a cell-free binding assay, which comprises
(1) a binding partner I which is a recombinant fusion protein composed of a receptor protein relevant for the binding to be investigated and of a carrier protein, where said fusion protein is coupled by means of an antiserum or monoclonal antibody, with retention of the binding activity of said receptor protein, to a suitable solid phase, being bound to
(a) a binding partner II which is a ligand, which is labeled in a manner suitable for measuring the binding to said receptor protein, for said receptor protein but is not an antibody and
(b) an unlabeled ligand, which is to be determined, for said receptor protein or a receptor protein, which is to be determined, in soluble form, and
(2) the amount of labeled natural or artificial ligands bound to the solid phase being detected.

25. The method as claimed in claim 24, wherein the unlabeled ligand to be determined or the soluble receptor protein to be determined is an antibody.

26. A process for the production of a binding assay as claimed in any of claims 1 to 17, which comprises coupling a recombinant fusion protein (binding partner I), composed of a receptor protein relevant for the binding to be investigated and of a carrier protein, to a suitable solid phase by means of an antiserum or monoclonal antibody, with retention of the binding activity of the receptor protein, where the other binding partner II, which is not an antibody, is labeled in a manner suitable for measuring the binding.

## Claims (Claims for the following Contracting State(s): ES, GR)

1. A method for the production of a cell-free binding assay which comprises coupling a recombinant fusion protein (binding partner I), composed of a receptor protein relevant for the binding to be investigated and of a carrier protein, to a suitable solid phase by means of an antiserum or monoclonal antibody, with retention of the binding activity of the receptor protein, where another binding partner II, which is a ligand for said receptor protein but is not an antibody is labeled in a manner suitable for measuring the binding to said receptor protein.

2. The method as claimed in claim 1, in which the receptor protein in the recombinant fusion protein is the extracellular domain of a cellular receptor or part thereof.

3. The method as claimed in claim 2, where the cellular receptor is a cytokine receptor.

4. The method as claimed in claim 2, where the cellular receptor is a growth factor receptor.

5. The method as claimed in claim 2, where the cellular receptor is a hormone receptor.

6. The method as claimed in claim 2, where the cellular receptor is a neurotransmitter receptor.

7. The method as claimed in claim 2, where the cellular receptor is the receptor for a pathogenic organism or part thereof.

8. The method as claimed in claim 1, in which the receptor protein in the recombinant fusion protein is the extracellular domain of a cell adhesion molecule or part thereof.

9. The method as claimed in claim 1, in which the receptor protein in the recombinant fusion protein is a soluble protein or part thereof.

10. The method as claimed in claim 1, in which the carrier protein in the recombinant fusion protein is an immunoglobulin molecule or part thereof.

11. The method as claimed in claim 10, in which the carrier protein in the recombinant fusion protein is the constant part of the heavy chain of an immunoglobulin or part thereof.

12. The method as claimed in claim 10 or 11, in which the carrier protein in the recombinant fusion protein is the constant part of the heavy chain of human IgG l or part thereof.

13. The method as claimed in claim 1 using a carrier protein in the recombinant fusion protein, in which the carrier protein does not influence the biological activity of the receptor protein and makes the fusion protein amenable to purification by affinity chromatography.

14. The method as claimed in any of claims 1 to 13, in which the binding partner II is radiolabeled.

15. The method as claimed in any of claims 1 to 13, in which the binding partner II is linked to a low molecular weight ligand (such as, for example, biotin) and can thus be detected in a further reaction step.

16. The method as claimed in any of claims 1 to 13, in which the binding partner II is linked to an enzymatically active protein (for example peroxidase, alkaline phosphatase, luciferase).

17. The method as claimed in claim 15, in which streptavidin coupled to peroxidase is used for the further reaction step.

18. The use of the binding assay as claimed in any of claims 1 to 17 for receptor screening for the identification of natural or synthetic agonists or antagonists of the particular interaction.

19. The use of the binding assay as claimed in any of claims 1 to 17 for the characterization of antibodies directed against one of the two binding partners.

20. The use of the binding assay as claimed in any of claims 1 to 17 for detecting the biological activity of soluble cellular receptors.

21. The use of the binding assay as claimed in any of claims 1 to 17 for detecting the biological activity of ligands.

22. The use of the binding assay as claimed in any of claims 1 to 17 for the functional analysis of ligands with specific modifications, or parts thereof.

23. The use of the binding assay as claimed in any of claims 1 to 17 for the identification of substances relevant to diagnosis or therapy.

24. A method for detecting the binding behavior of receptor proteins, which are normally in the cell membrane, toward natural or synthetic ligands in a cell-free binding assay, which comprises
(1) a binding partner I which is a recombinant fusion protein composed of a receptor protein relevant for the binding to be investigated and of a carrier protein, where said fusion protein is coupled by means of an antiserum or monoclonal antibody, with retention of the binding activity of said receptor protein, to a suitable solid phase, being bound to
(a) a binding partner II which is a ligand, which is labeled in a manner suitable for measuring the binding to said receptor protein, for said receptor protein but is not an antibody and
(b) an unlabeled ligand, which is to be determined, for said receptor protein or a receptor protein, which is to be determined, in soluble form, and
(2) the amount of labeled natural or artificial ligands bound to the solid phase being detected.

25. The method as claimed in claim 24, wherein the unlabeled ligand to be determined or the soluble receptor protein to be determined is an antibody.

## Revendications (Revendications pour l'(les) Etat(s) contractant(s) suivant(s): AT, BE, CH, DE, DK, FR, GB, IT, LI, LU, NL, SE)

1. Nécessaire pour la détection de liaison sans cellules, pour l'étude de la capacité de liaison de protéines récepteurs normalement stables dans les membranes cellulaires vis à vis de ligands naturels ou synthétiques, comprenant
(1) un partenaire de liaison I, qui est une protéine de fusion recombinante, constituée d'une protéine récepteur correspondant à la liaison étudiée, et d'une protéine support, ladite protéine de fusion étant couplée au moyen d'un antiserum ou d'un anticorps monoclonal à une phase solide appropriée, sans modification de l'activité de liaison de ladite protéine récepteur, et
(2) un partenaire de liaison II, qui n'est pas un anticorps mais qui est un ligand marqué pour ladite protéine récepteur, le marquage étant approprié à la mise en évidence d'une liaison à ladite protéine récepteur.

2. Nécessaire pour la détection de liaison selon la revendication 1, dans lequel la protéine récepteur dans la protéine de fusion recombinante est le domaine extracellulaire d'un récepteur cellulaire ou un fragment de celui-ci.

3. Nécessaire pour la détection de liaison selon la revendication 2, dans lequel le récepteur cellulaire est un récepteur de type cytokine.

4. Nécessaire pour la détection de liaison selon la revendication 2, dans lequel le récepteur cellulaire est un récepteur de type facteur de croissance.

5. Nécessaire pour la détection de liaison selon la revendication 2, dans lequel le récepteur cellulaire est un récepteur de type hormone.

6. Nécessaire pour la détection de liaison selon la revendication 2, dans lequel le récepteur cellulaire est un récepteur de type neurotransmetteur.

7. Nécessaire pour la détection de liaison selon la revendication 2, dans lequel le récepteur cellulaire est le récepteur pour un organisme pathogène ou un fragment de celui-ci.

8. Nécessaire pour la détection de liaison selon la revendication 1, dans lequel la protéine récepteur dans la protéine de fusion recombinante est le domaine extracellulaire d'une molécule d'adhésion cellulaire ou un fragment de celui-ci.

9. Nécessaire pour la détection de liaison selon la revendication 1, dans lequel la protéine récepteur dans la protéine de fusion recombinante est une protéine soluble ou un fragment de celle-ci.

10. Nécessaire pour la détection de liaison selon la revendication 1, dans lequel la protéine support dans la protéine de fusion recombinante est une molécule d'immunoglobuline ou un fragment de celle-ci.

11. Nécessaire pour la détection de liaison selon la revendication 10, dans lequel la protéine support dans la protéine de fusion recombinante est la région constante de la chaîne lourde d'une immunoglobuline ou un fragment de celle-ci.

12. Nécessaire pour la détection de liaison selon la revendication 10 ou 11, dans lequel la protéine support dans la protéine de fusion recombinante est la région constante de la chaîne lourde d'une IgG 1 humaine ou un fragment de celle-ci.

13. Nécessaire pour la détection de liaison selon la revendication 1, qui utilise une protéine support dans la protéine de fusion recombinante, dans lequel la protéine support n'influence pas l'activité biologique de la protéine récepteur et la protéine de fusion rend accessible une purification par chromatographie d'affinité

14. Nécessaire pour la détection de liaison selon les revendications 1 à 13, dans lequel le partenaire de liaison II est marqué par un élément radioactif.

15. Nécessaire pour la détection de liaison selon les revendications 1 à 13, dans lequel le partenaire de liaison II est lié à un ligand de faible poids moléculaire (comme p. ex. la biotine) et ainsi peut être décelé dans une étape ultérieure de réaction.

16. Nécessaire pour la détection de liaison selon les revendications 1 à 13, dans lequel le partenaire de liaison II est lié à une protéine enzymatique active (p. ex. une peroxydase, une phosphatase alcaline, une luciférase).

17. Nécessaire pour la détection de liaison selon la revendication 15, dans lequel on utilise, pour l'étape ultérieure de réaction, de la streptavidine couplée à une peroxydase.

18. Utilisation d'un nécessaire pour la détection de liaison selon les revendications 1 à 17 pour le criblage de récepteurs destinés à l'identification d'agonistes ou d'antagonistes naturels ou synthétiques de l'interaction concernée.

19. Utilisation d'un nécessaire pour la détection de liaison selon les revendications 1 à 17 pour la caractérisation d'anticorps dirigés contre l'un des deux partenaires de liaison.

20. Utilisation d'un nécessaire pour la détection de liaison selon les revendications 1 à 17 pour la détection de l'activité biologique de récepteurs cellulaires solubles.

21. Utilisation d'un nécessaire pour la détection de liaison selon les revendications 1 à 17 pour la détection de l'activité biologique de ligands.

22. Utilisation d'un nécessaire pour la détection de liaison selon les revendications 1 à 17 destiné à l'analyse fonctionnelle de ligands modifiés ou de fragments de ceux-ci.

23. Utilisation d'un nécessaire pour la détection de liaison selon l'une des revendications 1 à 17 pour l'identification de substances utilisables sur le plan diagnostique ou thérapeutique.

24. Procédé pour l'étude de la capacité de liaison de protéines récepteurs normalement stables dans les membranes cellulaires vis à vis de ligands naturels ou artificiels par un dosage de liaison exempt de cellules, caractérisé en ce que
(1) un partenaire de liaison I, qui est une protéine de fusion recombinante, constituée d'une protéine récepteur correspondant à la liaison étudiée, et d'une protéine support, ladite protéine de fusion étant couplée au moyen d'un antiserum ou d'un anticorps monoclonal à une phase solide appropriée sans modification de l'activité de liaison de ladite protéine récepteur, est lié à
(a) un partenaire de liaison II, qui n'est pas un anticorps mais qui est un ligand marqué pour ladite protéine récepteur, le marquage étant approprié à la mise en évidence d'une liaison à ladite protéine récepteur, et
(b) un ligand non marqué pour ladite protéine récepteur à déterminer, ou une protéine récepteur sous forme soluble à déterminer
et
(2) l'on détermine la quantité de ligand marqué naturel ou synthétique lié à la phase solide

25. procédé selon la revendication 24, caractérisé en ce que, le ligand non marqué à déterminer ou la protéine récepteur soluble à déterminer est un anticorps.

26. Procédé de fabrication d'un nécessaire pour la détection de liaison selon l'une des revendications 1 à 17, caractérisé en ce l'on l'on couple une protéine de fusion recombinante (partenaire de liaison I), constituée d'une protéine récepteur correspondant à la liaison étudiée, et d'une protéine support au moyen d'un antiserum ou d'un anticorps monoclonal à une phase solide appropriée sans modification de l'activité de liaison de la protéine récepteur, l'autre partenaire de liaison II, qui n'est pas un anticorps, étant marqué de manière appropriée pour la mise en évidence de la liaison.

## Revendications (Revendications pour l'(les) Etat(s) contractant(s) suivant(s): ES, GR)

1. Procédé de fabrication d'un nécessaire pour la détection de liaison sans cellules, caractérisé en ce que l'on couple une protéine de fusion recombinante (partenaire de liaison I), constituée d'une protéine récepteur correspondant à la liaison étudiée, et d'une protéine support au moyen d'un antiserum ou d'un anticorps monoclonal à une phase solide appropriée, sans modification de l'activité de liaison de la protéine récepteur, un autre partenaire de liaison II, qui n'est pas un anticorps mais est un ligand pour ladite protéine récepteur étant marqué de manière appropriée à la détection d'une liaison à ladite protéine récepteur,.

2. Procédé selon la revendication 1, dans lequel la protéine récepteur dans la protéine de fusion recombinante est le domaine extracellulaire d'un récepteur cellulaire ou un fragment de celui-ci.

3. Procédé selon la revendication 2, dans lequel le récepteur cellulaire est un récepteur de type cytokine.

4. Procédé selon la revendication 2, dans lequel le récepteur cellulaire est un récepteur de type facteur de croissance.

5. Procédé selon la revendication 2, dans lequel le récepteur cellulaire est un récepteur de type hormone.

6. Procédé selon la revendication 2, dans lequel le récepteur cellulaire est un récepteur de type neurotransmetteur.

7. Procédé selon la revendication 2, dans lequel le récepteur cellulaire est le récepteur pour un organisme pathogène ou un fragment de celui-ci.

8. Procédé selon la revendication 1, dans lequel la protéine récepteur dans la protéine de fusion recombinante est le domaine extracellulaire d'une molécule d'adhésion cellulaire ou un fragment de celui-ci.

9. Procédé selon la revendication 1, dans lequel la protéine récepteur dans la protéine de fusion recombinante est une protéine soluble ou un fragment du celle-ci.

10. Procédé selon la revendication 1, dans lequel la protéine support dans la protéine de fusion recombinante est une molécule d'immunoglobuline ou un fragment de celle-ci.

11. Procédé selon la revendication 10, dans lequel la protéine support dans la protéine de fusion recombinante est la région constante de la chaîne lourde d'une immunoglobuline ou un fragment de celle-ci.

12. Procédé selon la revendication 10 ou 11, dans lequel la protéine support dans la protéine de fusion recombinante est la région constante de la chaîne lourde d'une IgG 1 humaine ou un fragment de celle-ci.

13. Procédé selon la revendication 1, qui utilise une protéine support dans la protéine de fusion recombinante, dans lequel la protéine support n'influence pas l'activité biologique de la protéine récepteur et la protéine de fusion permet une purification par chromatographie d'affinité

14. Procédé selon les revendications 1 à 13, dans lequel le partenaire de liaison II est marqué de façon radioactive.

15. Procédé selon les revendications 1 à 13, dans lequel le partenaire de liaison II est lié à un ligand de faible poids moléculaire (comme p. ex. la biotine) et ainsi peut être décelé dans une étape ultérieure de réaction.

16. Procédé selon les revendications 1 à 13, dans lequel le partenaire de liaison II est lié à une protéine enzymatique active (p. ex. une peroxydase, une phosphatase alcaline, une luciférase).

17. Procédé selon la revendication 15, dans lequel on utilise, pour l'étape ultérieure de réaction, de la streptavidine couplée à une peroxydase.

18. Utilisation du test de liaison selon l'une des revendications 1 à 17 pour le criblage de récepteurs destinés à l'identification d'agonistes ou d'antagonistes naturels ou synthétiques de l'interaction concernée.

19. Utilisation du test de liaison selon l'une des revendications 1 à 17 pour la caractérisation d'anticorps dirigés contre l'un des deux partenaires de liaison.

20. Utilisation du test de liaison selon l'une des revendications 1 à 17 pour la détection de l'activité biologique de récepteurs cellulaires solubles.

21. Utilisation du test de liaison selon l'une des revendications 1 à 17 pour la détection de l'activité biologique de ligands.

22. Utilisation du test de liaison selon l'une des revendications 1 à 17 destiné à l'analyse fonctionnelle de ligands modifiés ou de parties de ceux-ci.

23. Utilisation du test de liaison selon l'une des revendications 1 à 17 pour l'identification de substances applicables sur le plan diagnostique ou thérapeutique.

24. Procédé pour l'étude de la capacité de liaison de protéines récepteurs normalement stables dans les membranes cellulaires vis à vis de ligands naturels ou synthétiques par mise en oeuvre d'un test de liaison sans cellules, caractérisé en ce que
(1) un partenaire de liaison I, qui est une protéine de fusion recombinante, constituée d'une protéine récepteur correspondant à la liaison étudiée, et d'une protéine support, ladite protéine de fusion est couplée au moyen d'un antiserum ou d'un anticorps monoclonal à une phase solide appropriée sans modification de l'activité de liaison de ladite protéine récepteur, est lié à
(a) un partenaire de liaison II, qui n'est pas un anticorps mais qui est un ligand marqué pour ladite protéine récepteur, de manière appropriée à la détection d'une liaison à ladite protéine récepteur,
(b) un ligand à déterminer non marqué, pour ladite protéine récepteur, ou une protéine récepteur sous forme soluble à déterminer
et
(2) l'on décèle la quantité de ligand marqué naturel ou synthétique lié à la phase solide

25. procédé selon la revendication 24, caractérisé en ce que, le ligand non marqué à déterminer ou la protéine récepteur soluble à déterminer est un anticorps.
